# EUROPÄISCHE PATENTSCHRIFT

(11) **EP 1 026 166 B1**
(45) Veröffentlichungstag und Bekanntmachung des Hinweises auf die Patenterteilung: **02.10.2002**
(21) Anmeldenummer: 00101922.3
(22) Anmeldetag: 01.02.2000
(51) Int. Cl.: C07D 475/04, C07C 49/175, C07C 251/08, C07C 237/36

(54) **Verfahren zur Herstellung von Folsäure**
Process for the preparation of folic acid
Procédé de préparation de l'acide folique

(30) Priorität: 05.02.1999 DE 19904812
(43) Veröffentlichungstag der Anmeldung: 09.08.2000
(73) Patentinhaber: BASF AKTIENGESELLSCHAFT, 67056 Ludwigshafen (DE)
(72) Erfinder: Botzem, Jörg, Dr., 67117 Limburgerhof (DE); Jaedicke, Hagen, Dr., 67069 Ludwigshafen (DE); John, Michael, Dr., 67245 Lambsheim (DE); Paust, Joachim, Dr., 67141 Neuhofen (DE)

(56) Entgegenhaltungen:
- EP-A- 0 122 479
- EP-A- 0 451 835
- EP-A- 0 472 118
- EP-A- 0 608 693
- WO-A-99/20626
- US-A- 2 436 073

## Beschreibung

Die vorliegende Erfindung betrifft ein neues Verfahren zur Herstellung von Folsäure.

Robert B. Angier et al. (JACS, 70 (1948), 25) beschreiben die Herstellung von Folsäure unter Einsatz von halogenfreien Verbindungen durch Umsetzung von p-Aminobenzoyl-L-glutaminsäurediethylester mit 2-Hydroxymalondiadehyd, Isolierung von p-(2,3-Dihydroxy-2-en-propylidenamino-)benzoesäurediethylester und Umsetzung des Zwischenprodukts mit Triaminopyrimidon.

Weitere Herstellmethoden sind beschrieben in: O. Isler, G. Brubacher, S. Ghisla, B. Kräutler, Vitamine II: G. Thieme Verlag Stuttgart (1988).

Allen diesen Methoden ist eine niedrige Ausbeute an Folsäure gemeinsam.

Weiterhin beschreibt EP-A-0 608 693 ein Verfahren zur Herstellung von Folsäure, bei dem aus 2-substitulerten Malondialdehyden durch Umsetzung mit p-Aminobenzoyl-L-glutaminsäure das entsprechende Diamin gebildet wird, welches mit Triaminopyrimidon in Gegenwart von Sulfit zu Folsäure umgesetzt wird. Nachteil dieses Verfahrens ist die schlechte Zugänglichkeit der 2-substituierten Malondialdehyde.

US 2,436,073 beschreibt ein Verfahren zur Herstellung von substituierten Pteridinen unter Verwendung von halogeniertem Methylglyoxaldialkylacetal.

Der vorliegenden Erfindung lag nun die Aufgabe zugrunde, ein Verfahren zur Herstellung von Folsäure bereitzustellen, mit dem Folsäure in guten Ausbeuten und unter Verwendung gut zugänglicher Einsatzstoffe erhalten wird.

Diese Aufgabe wurde gelöst durch ein Verfahren zur Herstellung von Folsäure, dadurch gekennzeichnet, daß man ein Tetraalkoxypropanol der allgemeinen Formel I, 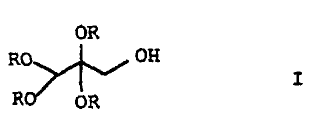 in der die Substituenten R für C₁-C₄-Alkyl stehen, mit Triaminopyrimidon der Formel II und p-Aminobenzoyl-L-glutaminsäure der Formel III 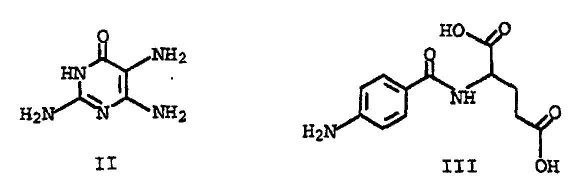 umsetzt.

Als Alkylreste für R sind C₁-C₄-Alkylreste, beispielsweise Methyl, Ethyl, n-Propyl, iso-Propyl oder n-Butyl, bevorzugt Methyl oder Ethyl, besonders bevorzugt Methyl gemeint.

Überraschenderweise wurde nun gefunden, daß durch Verwendung von Tetraalkoxypropanol der Formel I, insbesondere Tetramethoxypropanol, das durch elektrochemische Oxidation von Methylglyoxaldimethylacetal technisch gut zugänglich ist, Folsäure in hohen Ausbeuten erhalten werden kann.

Im Rahmen des erfindungsgemäßen Verfahrens kann dabei im ersten Reaktionsschritt die Umsetzung des Tetraalkoxypropanols sowohl mit Triaminopyrimidon der Formel II als auch mit der p-Aminobenzoyl-L-glutaminsäure der Formel III erfolgen, wobei die Reaktion vorteilhafterweise unter sauren Bedingungen bei pH-Werten kleiner als 4 und in einem Temperaturbereich von 0 bis 100°C ablauft.

Vorzugsweise erfolgt zunächst die Umsetzung des Tetraalkoxypropanols mit der p-Aminobenzoyl-L-glutaminsäure der Formel III.

Die Umsetzung kann in wäßrigem Medium, gegebenenfalls unter Zugabe von inerten, mit Wasser mischbaren, organischen Lösungsmitteln wie z.B. Acetonitril, Tetralhydrofuran, Dimethylformamid, Methanol, Ethanol etc. erfolgen.

Die sauren Reaktionsbedingungen lassen sich beispielsweise durch Zugabe von wäßrigen Mineralsäuren, wie z.B. wäßrige Salzsäure, Schwefelsäure, Phosphorsäure, durch Zugabe von organischen C₁-C₄-Carbonsäuren wie z.B. Ameisensäure, Essigsäure oder Propionsäure oder mittels eines sauren Ionenaustauschers einstellen. Bevorzugte Säuren sind dabei wäßrige Salzsäure, Ameisensäure und Essigsäure.

Unter den sauren Hydrolysebedingungen lassen sich aus den Verbindungen der allgemeinen Formel I in die entsprechenden Ketone der allgemeinen Formel la 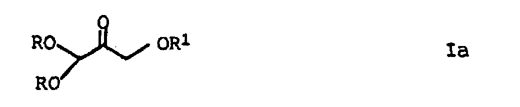 herstellen, in der die Substituenten R für C₁-C₄-Alkyl, R¹ für Wasserstoff oder -C(=O)-R² und R² für Wasserstoff oder C₁-C₃-Alkyl stehen.

Als Alkylreste für R sind die bereits o.g. C₁-C₄-Alkylreste gemeint.

Als Alkylreste für R² sind C₁-C₃-Alkylreste wie Methyl, Ethyl, n-Propyl oder iso-Propyl gemeint.

Bevorzugte Reste für R² sind Wasserstoff oder Methyl. Ganz besonders bevorzugt sind solche Ketone der Formel la, in der R für Methyl steht und R¹ Wasserstoff oder Acetyl [-C(=O)-CH₃] bedeutet.

Die eingangs genannte, bevorzugt durchgeführte Umsetzung von Tetraalkoxypropanol mit p-Aminobenzoyl-L-glutaminsäure der Formel III kann unter den o.g. sauren Bedingungen in situ über die Ketone der allgemeinen Formel la zu den Zwischenprodukten der allgemeinen Formel IV führen, 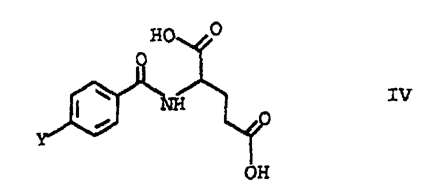 in der der Substituent Y 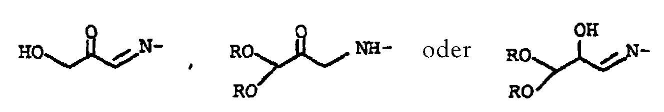 bedeutet und R für C₁-C₄-Alkyl, bevorzugt für Methyl steht

Die Umsetzung der Verbindungen der Formel IV mit Triaminopyrimidon der Formel II erfolgt vorteilhafterweise in Gegenwart von Sulfit, oder anorganischen Verbindungen, die in Wasser Sulfite bilden, bei einem pH-Wert von 3 bis 8 und Temperaturen von etwa 0 bis 100°C.

Als Sulfite oder anorganische Verbindungen, die in Wasser Sulfite bilden, werden Verbindungen wie z.B. Na₂SO₃, K₂SO₃, H₂SO₃, NaHSO₃, Na₂S₂O₅ oder SO₂ und dergleichen eingesetzt Ebenso ist de3r Einsatz von Triaminopyrimidon-Sulfit möglich.

Die bei der erfindungsgemäßen Herstellung der Folsäure gebildeten Zwischenprodukte la und IV können nach ihrer Herstellung isoliert und dann weiter in dem erfindungsgemäßen Verfahren eingesetzt werden. Sie können jedoch auch in situ hergestellt werden, und die Umsetzung kann demnach auch in einer Eintopfreaktion durchgeführt werden.

Gegenstand der Erfindung sind auch Ketone der allgemeinen Formel la, 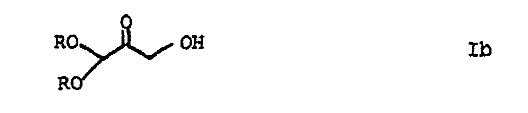 in der der Substituent R für C₁-C₄-Alkylsteht.

Bevorzugt sind Ketone der allgemeinen Formel Ib, in der der Substituent R für Methyl steht.

Gegenstand der Erfindung sind ebenfalls Verbindungen der allgemeinen Formel IV, 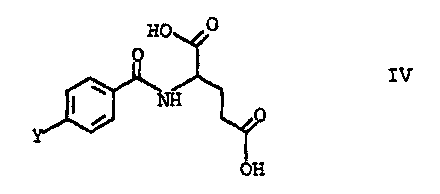 in der der Substituent Y 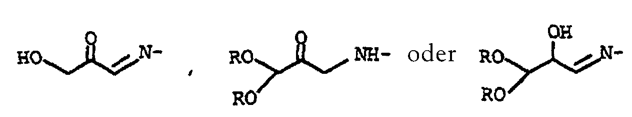 bedeutet und R für C₁-C₄-Alkyl, bevorzugt für Methyl steht.

### Beispiel

7,5 g 2,2,3,3-Tetramethoxypropan-1-ol wurden in 25 ml Essigsäure gelöst und auf Rückfluß erhitzt. Man destillierte ca. 1,5 g eines Leichtsieders ab. Nach zweistündiger Reaktionszeit wurde bei 20 mbar destilliert. Bei 110 bis 120°C destillierten 5,8 g einer Fraktion von ca. 80% Acetoxymethylglyoxaldimethylacetal (A) und 12 % Hydroxymethylglyoxaldimethylacetal (B).

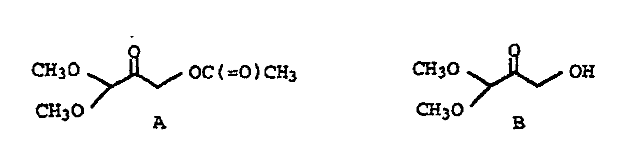

## Patentansprüche

1. Verfahren zur Herstellung von Folsäure, **dadurch gekennzeichnet, daß** man ein Tetraalkoxypropanol der allgemeinen Formel I, 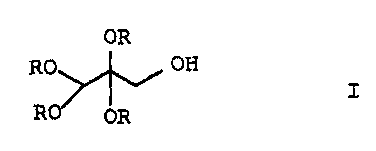 in der die Substituenten R für C₁-C₄-Alkyl stehen, mit Triaminopyrimidon der Formel II und p-Aminobenzoyl-L-glutaminsäure der Formel III 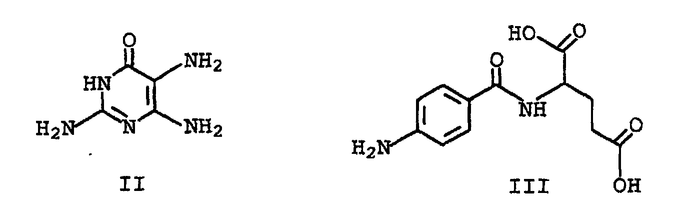 umsetzt.

2. Verfahren nach Anspruch 1, **dadurch gekennzeichnet, daß** man zunächst Tetraalkoxypropanol der allgemeinen Formel I mit p-Aminobenzoyl-L-glutaminsäure der Formel III umsetzt und das gebildete Reaktionsprodukt anschließend mit Triaminopyrimidon der Formel II zur Folsäure reagieren läßt.

3. Verfahren nach Anspruch 1, **dadurch gekennzeichnet, daß** man zunächst Tetraalkoxypropanol der allgemeinen Formel I mit Triaminopyrimidon der Formel II umsetzt und das gebildete Reaktionsprodukt anschließend mit p-Aminobenzoyl-L-glutaminsäure der Formel III zur Folsäure reagieren läßt.

4. Verfahren nach Anspruch 1, **dadurch gekennzeichnet, daß** das Verfahren als Eintopfreaktion geführt wird.

5. Verfahren nach einem der Ansprüche 1 bis 4, **dadurch gekennzeichnet, daß** die Umsetzung mit Triaminopyrimidon in Gegenwart von Natriumsulfit erfolgt.

6. Verfahren nach einem der Ansprüche 1 bis 5, **dadurch gekennzeichnet, daß** man das Tetraalkoxypropanol der allgemeinen Formel I vor der Umsetzung mit den Verbindungen der Formeln II und/oder III sauer hydrolysiert.

7. Verfahren nach Anspruch 6, **dadurch gekennzeichnet, daß** man das Tetraalkoxypropanol der allgemeinen Formel I vor der Umsetzung mit den Verbindungen der Formeln II und/oder III in Gegenwart einer C₁-C₄-Carbonsäure zum Keton der allgemeinen Formel Ia hydrolysiert, 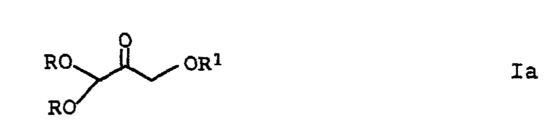 in der die Substituenten R für C₁-C₄-Alkyl, R¹ für Wasserstoff oder -C(=O)-R² und R² für Wasserstoff oder C₁-C₃-Alkyl stehen.

8. Verfahren nach einem der Ansprüche 1 bis 7, **dadurch gekennzeichnet, daß** man Tetramethoxypropanol mit Triaminopyrimidon der Formel II und p-Aminobenzoyl-L-glutaminsäure der Formel III umsetzt.

9. Verbindungen der allgemeinen Formel IV, 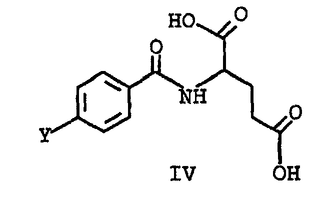 in der der Substituent Y 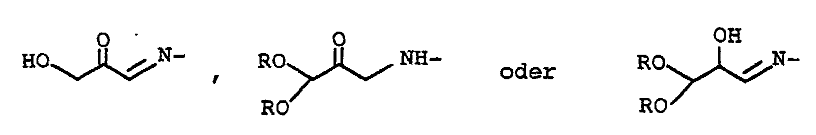 bedeutet und R für C₁-C₄-Alkyl steht.

## Claims

1. A process for preparing folic acid, which comprises reacting a tetraalkoxypropanol of the general formula I, 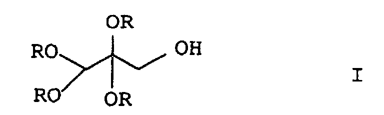 in which the substituents R are C₁-C₄-alkyl, with triaminopyrimidone of the formula II and p-aminobenzoyl-L-glutamic acid of the formula III

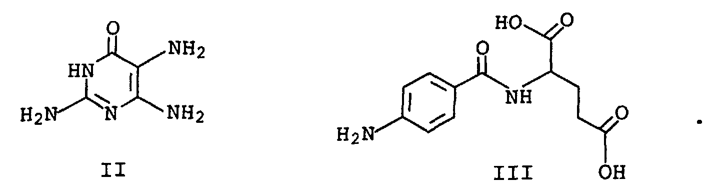

2. A process as claimed in claim 1, wherein firstly tetraalkoxypropanol of the general formula I is reacted with p-aminobenzoyl-L-glutamic acid of the formula III, and subsequently the product formed in the reaction is reacted with triaminopyrimidone of the formula II to give folic acid.

3. A process as claimed in claim 1, wherein firstly tetraalkoxypropanol of the general formula I is reacted with triaminopyrimidone of the formula II, and subsequently the product formed in the reaction is reacted with p-aminobenzoyl-L-glutamic acid of the formula III to give folic acid.

4. A process as claimed in claim 1, which is carried out as a one-pot reaction.

5. A process as claimed in any of claims 1 to 4, wherein the reaction with triaminopyrimidone takes place in the presence of sodium sulfite.

6. A process as claimed in any of claims 1 to 5, wherein the tetraalkoxypropanol of the general formula I is hydrolyzed with acid before the reaction with the compounds of the formulae II and/or III.

7. A process as claimed in claim 6, wherein the tetraalkoxypropanol of the general formula I is hydrolyzed before the reaction with the compounds of the formulae II and/or III in the presence of a C₁-C₄-carboxylic acid to give the ketone of the general formula Ia 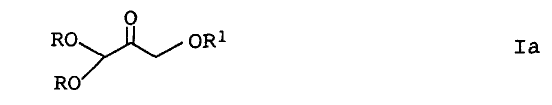 in which the substituents R are C₁-C₄-alkyl, R¹ is hydrogen or -C(=O)-R² and R² is hydrogen or C₁-C₃-alkyl.

8. A process as claimed in any of claims 1 to 7, wherein tetramethoxypropanol is reacted with triaminopyrimidone of the formula II and p-aminobenzoyl-L-glutamic acid of the formula III.

9. A compound of the formula IV 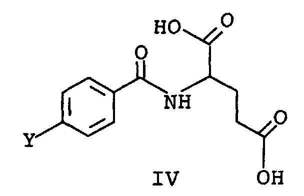 in which the substituent Y is 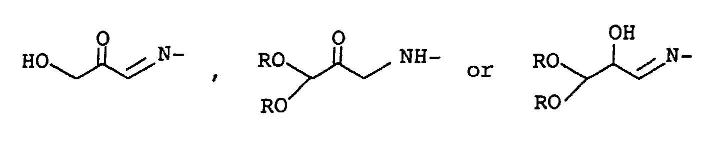 and R is C₁-C₄-alkyl.

## Revendications

1. Procédé pour la préparation d'acide folique, **caractérisé par le fait qu'**on fait réagir un tétraalcoxypropanol de formule générale I, 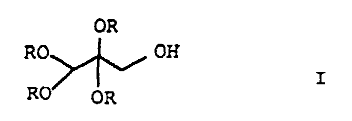 dans laquelle les substituants R désignent un alkyle en C₁-C₄, avec de la triaminopyrimidone de formule II et de l'acide p-aminobenzoyl-L-glutamique de formule III

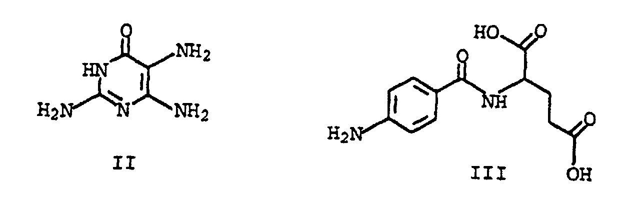

2. Procédé selon la revendication 1, **caractérisé par le fait qu'**on fait d'abord réagir le tétraalcoxypropanol de formule générale I avec l'acide p-aminobenzoyl-L-glutamique de formule III et on fait ensuite réagir le produit de réaction formé avec la triaminopyrimidone de formule II pour donner l'acide folique.

3. Procédé selon la revendication 1, **caractérisé par le fait qu'**on fait d'abord réagir le tétraalcoxypropanol de formule générale I avec la triaminopyrimidone de formule II et on fait ensuite réagir le produit de réaction formé avec l'acide p-aminobenzoyl-L-glutamique de formule III pour donner l'acide folique.

4. Procédé selon la revendication 1, **caractérisé par le fait qu'**on conduit la réaction dans un récipient unique.

5. Procédé selon l'une des revendications 1 à 4, **caractérisé par le fait que** la réaction est effectuée avec de la triaminopyrimidone en présence de sulfite de sodium.

6. Procédé selon l'une des revendications 1 à 5, **caractérisé par le fait qu'**on soumet à une hydrolyse acide le tétraalcoxypropanol de formule générale I avant la réaction avec les composés de formules II et/ou III.

7. Procédé selon la revendication 6, **caractérisé par le fait qu'**on hydrolyse le tétraalcoxypropanol de formule générale I avant la réaction avec les composés de formules II et/ou III en présence d'un acide carboxylique en C₁-C₄ pour former la cétone de formule générale Ia, 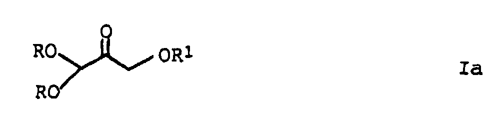 dans laquelle les substituants R désignent des groupes alkyle en C₁-C₄, R¹ représente l'hydrogène ou -C(=O)-R² et R² désigne l'hydrogène ou un groupe alkyle en C₁-C₃.

8. Procédé selon l'une des revendications 1 à 7, **caractérisé par le fait qu'**on fait réagir du tétraméthoxypropanol avec de la triaminopyrimidone de formule II et de l'acide p-aminobenzoyl-L-glutamique de formule III.

9. Composés de formule générale IV, 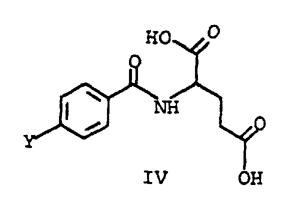 dans laquelle le substituant Y désigne 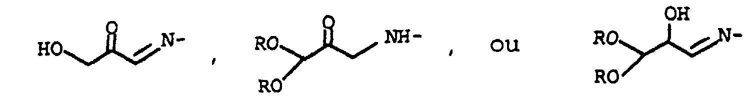 et représente un groupe alkyle en C₁-C₄.
